Europäisches Patentamt

European Patent Office

Office européen des brevets

(11) Publication number : **0 404 162 B1**

# EUROPEAN PATENT SPECIFICATION

(45) Date of publication of patent specification :
24.02.93 Bulletin 93/08

(51) Int. Cl.⁵ : **A61K 7/48,** A61K 31/35

(21) Application number : **90111782.0**

(22) Date of filing : **21.06.90**

(54) Composition for treating pigmentation.

The file contains technical information
submitted after the application was filed and
not included in this specification

(30) Priority : **23.06.89 JP 161155/89**

(43) Date of publication of application :
**27.12.90 Bulletin 90/52**

(45) Publication of the grant of the patent :
**24.02.93 Bulletin 93/08**

(84) Designated Contracting States :
**CH DE FR GB IT LI NL SE**

(56) References cited :
**EP-A- 0 289 027
WO-A-88/09659
PATENT ABSTRACTS OF JAPAN, unexamined
applications, C field, vol. 12, no. 209, June 15,
1988 THE PATENT OFFICE
JAPANESEGOVERNMENT page 91 C 504**

(56) References cited :
**PATENT ABSTRACTS OF JAPAN, unexamined
applications, C field, vol. 12, no. 209, June 15,
1988 THE PATENT OFFICE
JAPANESEGOVERNMENT page 91 C 504
CHEMICAL ABSTRACTS, vol. 101, no. 20,
November 12, 1984 Columbus, Ohio, USA H.
YOSHITAKA "Inhibitory effect of kojic acid
onmelanogenesis" page 372, column 1, ab-
stract no. 177 254r**

(73) Proprietor : **Sansho Seiyaku Co., Ltd.
26-7, Oike 2-chome
Onojo-shi Fukuoka-ken (JP)**

(72) Inventor : **Mishima, Yutaka
4-32, Sowa-machi 1-chome, Nada-ku
Kobe-shi, Hyogo-ken (JP)**
Inventor : **Motono, Masahiro
272-5, Nishi-machi
Kurume-shi, Fukuoka-ken (JP)**

(74) Representative : **Müller-Boré & Partner
Patentanwälte
Isartorplatz 6 Postfach 26 02 47
W-8000 München 2 (DE)**

EP 0 404 162 B1

## Description

BACKGROUND OF THE INVENTION

Field of the Invention

The present invention relates to a medicament for safely and effectively treating pigmentation diseases such as senile moles, sunlight induced moles, pigmented spots after inflammation, or chloasma not attributable to exogenous stimulation such as sunburn, which have been regarded as refractory diseases.

Prior Art

As applications to the skin for reducing freckles, etc. generated on the skin surface attributable to exogenous stimulation such as sunburn, the use of bleachable substances such as hydroquinone and melanogenesis inhibitors such as kojic acid is heretofore known. For example, kojic acid used as melanogenesis inhibitor is disclosed in Japanese Patent No. 1,352,153.

However, no effective agent for the treatment of senile moles, sunlight-induced moles, pigmented spots after inflammation, endogenous chloasma not attributable to exogenous stimulation such as sunburn, is known yet.

The object of the present invention is to provide medicaments for effectively treating senile moles, sunlight-induced moles, pigmented spots after inflammation, or endogenous chloasma not attributable to exogenous stimulation such as sunburn, etc, which have been regarded as refractory diseases with no known therapeutic agents up to now.

The present inventors have worked to solve the problems described above and have found that kojic acid used for inhibiting the formation of melanin such as chloasma, freckles, caused by exogenous stimulation such as ultraviolet rays, is extremely effective to cure diseases such as senile moles, sunlight-induced moles, pigmented spots after inflammation and endogenous chloasma, all of whose generating mechanisms are quite different; and, thus, the present invention has been accomplished.

SUMMARY OF THE INVENTION

The present invention relates to the use of kojic acid for the manufacture of a medicament for the treatment of endogenous chloasma, senile moles, sunlight-induced moles or pigmented spots after inflammation.

DETAILED DESCRIPTION OF PREFERRED EMBODIMENTS

In senile moles, which develop with the aging of the skin, and sunlight-induced moles, caused by exposure to excessive sunlight, which are subject of the treatment of the claimed composition, the conditions become worse as the count of melanocyte in the skin increases, and these diseases might sometimes ultimately be transformed into cancer. Pigmented spots after inflammation are refractory blackish brown pigmented spots that remaine after cure of some skin diseases.

Endogenous chloasma are not exogenous liver spots caused by ordinary sunburn but endogenous liver spots attributed to an abnormality in hormone metabolism or administration of various medicaments for the treatment of organ diseases, and are refractory pigmental degenerations.

Kojic acid of the present invention is a substance obtained by culturing the bacteria belonging to the genus Aspergillus, the genus Escharichia, the genus Actobacter, the genus Gluconobacter, and is 5-oxy-2-oxymethyl-$\gamma$-pyrone.

This substance has the effect of inhibiting melanin formation and has been used such as for whitening cosmetics.

The composition of the present invention is applied to the affected part mainly by percutaneous administration to the skin. Therefore, the composition is prepared in the form of ointment, liquid, lotion, cream, or aerosol. In the present invention, agents, additives and extenders. conventionally used for the preparations, are used in a conventional manner to obtain the preparations for percutaneous administration.

A content of kojic acid which is the effective component contained in the preparation for percutaneous administration is 0.1 to 5.0 % (by weight).

When using the preparation for percutaneous administration, the preparation is sufficiently applied to the affected part twice a day (in the morning and at night).

The toxicity of kojic acid, which is the effective ingredient of the present invention, is extremely low; $LD_{50}$

is 2.050 mg/kg in mice and 2.260 mg/kg in rats, by subcutaneous administration. Therefore, the present invention is quite safe as a medicament.

Next, the results of the composition of the present invention on clinical tests are shown below with respect to therapeutic effects to endogenous chloasma, senile moles and pigmented spots after inflammation.

Clinical Test:

(1) Sample

Cream obtained in Example 6

(2) Patients applied

Patients under consultation at university hospitals: endogenous chloasma, 62 patients; senile moles, 24 patients; and pigmented spots after inflammation, 22 patients.

(3) Method of Test

The sample was sufficiently applied to the left area of the affected part on the faces of the patients in a dose of 0.5 g each twice a day (in the morning and at night). As a control for the test, the right area of each affected part remained untreated.

(4) Evaluation

After application, the left area (applied part) was compared with the right area (unapplied part) with passage of time and the condition of cure was visually evaluated.

The criteria for evaluation are as follows:

Markedly effective:     The pigmented spots almost disappeared
Effective:              Judged to be considerably effective
Somewhat effective:     Pigmentation was slightly reduced

(5) Result of Test

The results are shown in Table 1 described below.

Table 1

| Disease | Markedly Effective | Effective | Somewhat Effective | Not Effective | Side Effect | Total | Effective Rate (%) |
|---|---|---|---|---|---|---|---|
| Endogenous Chloasma | 7 | 31 | 11 | 10 | 3 | 62 | 79.0 |
| Senile Moles | 4 | 6 | 2 | 10 | 0 | 24 | 58.3 |
| Pigmented Spots after Inflammation | 4 | 12 | 4 | 2 | 0 | 22 | 88.8 |
| Total | 15 | 49 | 17 | 22 | 3 | 108 | |

Numerals in the Table show the number of patients.

In the therapeutic period of the above test, the effects revealed in one month at the earliest and four months at the latest.

[Examples]

Example 1: Liquid

| | |
|---|---|
| Kojic acid | 2.0 (%) |
| Glycerine | .0 (%) |
| Sorbitol | 4.0 (%) |
| Polyoxy stearate 40 | 1.55 (%) |
| Ethanol | 10.0 (%) |
| Sodium hydrogensulfite | 0.05 (%) |
| Disodium EDTA | 0.02 (%) |
| Sodium glutamate | 0.5 (%) |
| Purified water | making up 100 (%) |

The components are mixed with stirring and dissolved to obtain the liquid.

Example 2: Emulsified lotion

A:

Kojic acid                    2.5 (%)

Propylene glycol              4.0 (%)

Ascorbic acid                 1.0 (%)

Purified water      making up 100 (%)


B:
Soy bean lecithin          0.8 (%)
dl-α-Tocopherol            0.1 (%)
Ethanol                    5.0 (%)
Polyethylene glycol 400    6.0 (%)
Purified water             20.0 (%)
C:
Purified water             1.0 (%)
Disodium EDTA              0.02 (%)
Potassium hydrogentartrate 0.2 (%)

The components of A, B and C are mixed with stirring and dissolved to obtain the emulsified lotion.


Example 3: Oleophilic ointment


A:
Kojic acid        3.0 (%)
Polysorbate 20    5.0 (%)
Dextran           0.07 (%)
Ethanol           5.0 (%)
B:


White vaseline      making up 100 (%)

Tallow                       20.0 (%)

Cetanol                       2.0 (%)

dl-α-Tocopherol               0.1 (%)

Glycerine monostearate        5.0 (%)


The components of A are mixed. The components of B are mixed and stirred with heating. A is mixed with the mixture of B upon cooling to obtain the oleophilic ointment.


Example 4: Water-Soluble Ointment


A:
Carboxyvinyl polymer    1.0 (%)
Propylene glycol        10.0 (%)
Ethanol                 8.0 (%)
Diisopropanol amine     0.15 (%)
B:
Triacetyne      30.0 (%)
Xanthane gum    2.0 (%)
C:
Purified water          3.0 (%)
Sodium hydrogensulfite  0.03 (%)
D:

Purified water     10.0 (%)
Kojic acid       1.5 (%)

The components of A are mixed to form a gel. Then, B, C and D are successively added to A to obtain the water-soluble ointment.

Example 5: Emulsified ointment

| | |
|---|---|
| White vaseline | 25.0 (%) |
| Silicone oil | 5.0 (%) |
| Stearyl alcohol | 22.0 (%) |
| Propylene glycol | 12.0 (%) |
| Sucrose fatty acid ester | 5.0 (%) |
| Polyoxyl stearate 40 | 2.5 (%) |
| dl-α-Tocopherol | 0.15 (%) |
| Kojic acid | 5.0 (%) |
| Purified water | making up 100 (%) |

The components are homogeneously mixed with heating and cooled to obtain the emulsified ointment.

Example 6: Cream

A:

| | |
|---|---|
| Purified water | making up 100 (%) |
| 1,3-Butylene glycol | 3.0 (%) |
| Sorbitol | 7.0 (%) |
| Sodium dl-PCA (50 % solution) | 3.0 (%) |
| Carboxyvinyl polymer | 0.05 (%) |

B:
Polysorbate 60         2.5 (%)
Monostearic acid glyceride  1.5 (%)
Cetanol                3.0 (%)
Vaseline              5.0 (%)
Octydodecyl myristate     5.0 (%)
Octyl dodecanol        6.0 (%)
Squalane            11.0 (%)
dl-α-Tocopherol       0.15 (%)
C:
Purified water    10.0 (%)
Kojic acid       2.0 (%)

A and B are heated to dissolve, respectively. B is added to A to emulsify and cooled to prepare the cream.

Separately, C is dissolved and added to the cream to obtain the cream composition.

Example 7: Aerosol

| | |
|---|---|
| Kojic acid | 2.0 (%) |
| Cetanol | 1.2 (%) |
| Propylene glycol | 4.0 (%) |
| Stearic acid | 8.0 (%) |
| Purified water | making up 100 (%) |
| Fulon 123/141b (57:43) | 7.0 (%) |

The components are mixed and dissolved. The solution is put in an aerosol container to obtain the aerosol.

Example 8: Cataplasm

A:

| | |
|---|---|
| Polyacrylic acid | 30.0 (%) |
| Sorbitan monooleate | 1.0 (%) |
| Purified water | making up 100 (%) |

B:

| | |
|---|---|
| Sodium polyacrylate | 7.0 (%) |
| Aluminum chloride | 0.3 (%) |
| Concentrated glycerin | 20.0 (%) |
| Titanium oxide | 4.0 (%) |
| Kojic acid | 5.0 (%) |

A and B are heated to dissolve, respectively. B is added to A, homogenously stirred and cooled. The resultant mixture is spread on a backing to obtain the cataplasm.

Example 9: Plaster

A:

| | |
|---|---|
| Polystyrene-polyisoprene-polystyrene rubber | 40.0 (%) |
| Liquid paraffin | 25.0 (%) |

B:

| | |
|---|---|
| Ester gum | 15.0 (%) |
| Polybutene | 15.0 (%) |
| Butylhydroxytoluene | 1.0 (%) |

C:

| | |
|---|---|
| Kojic acid | 4.0 (%) |

A is heated to dissolve, to which B is gradually added and stirred homogenously. Then, C is added to the mixture and spread on a backing to obtain the plaster.

7

In the Examples, percent is by weight.

The present invention provides the extremely effective medicaments for the treatment of pigmentation such as endogenous chloasma, senile moles, sunlight-induced moles, pigmented spots after inflammation, etc., which have been heretofore difficult to cure and the aggravation of which causes serious conditions. Further, no side effects are observed in administering and, therefore, the compositions of the present invention are quite safe and effective medicaments.

## Claims

1. Use of kojic acid for the manufacture of a medicament for the treatment of endogenous chloasma, senile moles, sunlight-induced moles or pigmented spots after inflammation.

## Patentansprüche

1. Verwendung von Kojisäure zur Herstellung eines Arzneimittels zur Behandlung von endogener Chloasma, altersbedingter Flecken, Sonnenlicht-induzierter Flecken oder Pigmentflecken nach Entzündung.

## Revendications

1. Utilisation de l'acide kojique pour la fabrication d'un médicament pour le traitement d'un chloasma endogène, de naevi séniles, de naevi provoqués par la lumière du soleil ou de taches pigmentées après une inflammation.